(19) Europäisches Patentamt
European Patent Office
Office européen des brevets

(11) Publication number: **0 332 946 B1**

(12) **EUROPEAN PATENT SPECIFICATION**

(45) Date of publication of patent specification: **12.05.93**

(51) Int. Cl.[5]: **C07K 5/06**, A61K 37/02

(21) Application number: **89103699.8**

(22) Date of filing: **03.03.89**

(54) **The dipeptide L-pyroglutamyl-L-cysteine, having a high anti-cataractogenic activity, and pharmaceutical compositions containing the same.**

(30) Priority: **15.03.88 IT 1978388**

(43) Date of publication of application:
**20.09.89 Bulletin 89/38**

(45) Publication of the grant of the patent:
**12.05.93 Bulletin 93/19**

(84) Designated Contracting States:
**AT BE CH DE ES FR GB GR IT LI LU NL SE**

(56) References cited:
**GB-A- 2 156 818**

**CHEMICAL ABSTRACTS, vol. 91, no. 3, 16th July 1979, page 718, column 1, abstract no. 21136t; & JP-A-54 19966 (SHOWA DENKO K.K.), 15-02-1979 in combination with CHEMICAL SUBSTANCE INDEX CH. lo, July-December 1979, page 1916, column 1, line 116**

**CHEMICAL ABSTRACTS, vol. 96, no. 24, 14th June 1982; page 379, column 1, abstract no. 20541n; & JP-A-57 19615 (SENJU PHARMACEUTICAL CO. LTD.)**

(73) Proprietor: **S.I.F.I. Società Industria Farmaceutica Italiana S.p.A.
Via Nicola Coviello 15/B
I-95128 Catania(IT)**

(72) Inventor: **Benanti, Giuseppe, Dr.
Via Barriera del Bosco, 9/A
I-95030 S. Agata Li Battiati (Catania)(IT)**
Inventor: **Rizza, Victor, Prof.
Contrada Ognina
I-96100 Siracusa(IT)**

(74) Representative: **Giambrocono, Alfonso, Dr. Ing. et al
Ing. A. Giambrocono & C. S.r.l. Via Rosolino Pilo 19/B
I-20129 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention relates to the dipeptide L-pyroglutamyl-L-cysteine of the formula

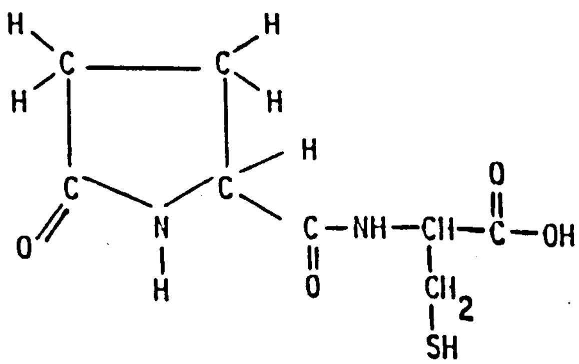

for use as an active therapeutic substance.

The dipeptide of the present invention (hereinafter referred to as PGC) develops remarkable effects in the prevention and the therapy of the cataract. Said effects have both an experimental and clinical value.

Pyroglutamic acid (pyrrolidone-carboxylic acid, PCA) is a cyclic lactame derivative of the glutamic acid that occupies the N-terminal position of several peptides and proteins and is ubiquitously distributed in the body cells and fluids. Recent studies have shown that the formation of PCA in vivo is associated with the sinthesis of glutathione (GSH) through the gama-glutamic acid cycle (Robins R.J., Davies D.D.: (1981) Biochem. J. 19, 63-70; Meister A. : (1973 Science 180, 33-39). It has moreover been demonstrated that PCA increases the intracellular glutathione concentration, thus developing an intensive antioxidant activity by means of a protective action of the thiol groups (Wellner V.P., Sekura R., Meister A., Larson A.: (1974) Proc. Nat. Acad. Sci. USA, 71, 2505-2509). Moreover, PCA exhibits both in vivo and in vitro an ammonium fixing and haemoglobinemia antagonizing action that is manifested through a reduction of the levels and velocity of metahaemoglobine formation (Fatini G., Somigli M., Montagnani L.: (1964) Gazz. Med. Ita., Vol. 123, N 11, 344-348; Ciaceri G.: (1965) Giorn. biochem., Vol. XIV, N5, 347-354).

Glutathione can be synthesized in various kind of tissues, such as liver, intestine, erythrocytes and muscle. The synthesis occurs in two steps in which glutamate, glycine and cysteine are transformed into glutathione, said steps being catalized by the enzyme gama-glutamyl-cysteine synthetase and the enzyme glutathione synthetase that carry out a six-reaction cycle, the gama-glutamic cycle, that describes the degradation and resynthesis of glutathione. The former catalyzes the copulation of cysteine with glutamate to form gama-glutamyl- cysteine in the presence of ADP and inorganic phosphate; the glutathione synthetase enzyme yields eventually the complete form of glutathione by coupling glicine with gama-glutamyl-cysteine.

The above-mentioned features of PCA appear to be extremely important for a possible anticataractogenic activity thereof. In fact, the cataract pathogenesis seems to be bound with a hyperoxidation of the soluble proteins normally present in the lens and with their ensuing denaturation and precipitation. Said insoluble protein deposit in the lens fibers causes it to loose its transparence. The main antioxidant mechanism in the lens is based on glutathione, the concentration of which is strongly lowered in the lens affected by cataract (Dische Z., Zil H.: (1950) Am. J. Ophthalmol., 34, 104; Vaughan D., Ashbury T.: (1978) "Manuale di Oculistica", Piccin Ed. Padova.). The PCA, through its antioxidant action at the tissue level, which is mediated by an increased glutathione synthesis, has therefore the characteristics of an effective substance in preventing the appearance of the lens opaqueness or in causing it to regress.

The L-pyroglutamy-L-cysteine dipeptide has been described for the first time in Japanese Kokai Tokkyo Koho JP 79/19966 but no therapeutic activity thereof was suggested.

It has beeen now surprisingly found that L-pyroglutamyl-L-cysteine is endowed with therapeutic activity, in particular anticataractogenic activity.

Compounds having anticataractogenic properties are indeed per se well known, such as the ones disclosed in Japanese Kokai Tokkyo Koho JP 82/18615 which are salts of cysteine glucuronic acid, but such compounds have completely different structures which do not make obvious the structure of the pro sent application.

The present invention therefore relates to PGC dipeptide for use as a therapeutically active substance, in particular as anticataractogenic agent.

Of course the invention concerns also a medical preparation containing such dipeptide, especially for anticataractogenic use.

In the dipeptide PGC of the present invention, also the amino-acid residue of the cysteine can develop an anticataractogenic activity. It has been proved, in fact, that the cysteine content in the normal lens is much higher than that of cystine and that the oxidation-reduction balance of the two amino-acids is shifted towards cystine in the lens affected by cataract. This suggests that a high cysteine content within the lens can perform an inhibiting action on the cataract formation.

PGC, that had been synthesized according to the known method of the art, shows remarkable effects, that are provided with experimental and clinical value, on the formation of cataract. In the following the pharmacological effects of PGC on cataract models in vivo and in vitro and the toxicology of the substance are described.

## PHARMACOLOGY

### IN VIVO EXPERIMENTAL CATARACT MODELS

a) Cataract from tryptophan deficiency

Male albino Wistar rats with an average weight of 180 g have been used. Said rats have been fed a synthetic diet devoid of tryptophan for a 30 days period and divided in three groups: the first animal group has been administered PGC subcutaneously at a dosage of 1 mg/kg in buffer solution, the second group has been administered a 10 mg/kg doses, whilst the animals of the third group have been injected only buffer solution.

The conditions of the various eye sections :
conjunctiva, cornea, interior chamber, lens and vitreous humor have been evaluated at the slit lamp.

Especially the localisation and the degree of lens opaqueness have been scrutinized and documented by means of photographs.

Table 1 shows the results of the PGC activity upon the cataract of rats that have been treated with a tryptophan-deficient diet. It has been possible to observe that the control rats exhibited a certain amount of lens opaqueness after a thirty days treatment, whilst in the animals treated with PGC, both at 1 and at 10 mg/kg, the lens was perfectly transparent. It was possible to observe opaqueness predominantly at the level at the posterior capsule and there was also evidence of an initial sclerosis of the nucleus. No further alterations either at the level of the anterior segment or of the posterior segment have been observed.

TABLE 1

Activity of the dipeptide pyroglutamyl-cysteine (PGC) on the cataract induced by a tryptophan-deficient diet.

| Progress from treatment beginning (days) | | 10 | 20 | 30 |
|---|---|---|---|---|
| lens opaqueness | | | | |
| controls (8) | RE | - | - | +- |
| | | - | - | ++ |
| | | - | - | ++ |
| | | - | - | ++ |
| | | - | - | ++ |
| | | - | - | ++ |
| | | - | - | ++ |
| | | - | - | ++ |
| | LE | - | - | ++ |
| | | - | - | ++ |
| | | - | - | ++ |
| | | - | - | ++ |
| | | - | - | ++ |
| | | - | - | ++ |
| | | - | - | ++ |
| | | - | - | ++ |
| PGC 1mg/kg (8) | RE | - | - | - |
| | | - | - | - |
| | | - | - | - |
| | | - | - | - |
| | | - | - | - |
| | | - | - | - |
| | | - | - | - |
| | | - | - | - |
| | LE | - | - | - |
| | | - | - | - |
| | | - | - | - |
| | | - | - | - |
| | | - | - | - |
| | | - | - | - |
| | | - | - | - |
| | | - | - | - |
| PGC 10 mg/kg (8) | | - | - | - |
| | | - | - | - |
| | | - | - | - |
| | | - | - | - |
| | | - | - | - |
| | | - | - | - |
| | | - | - | - |
| | | - | - | - |
| | LE | - | - | - |
| | | - | - | - |
| | | - | - | - |
| | | - | - | - |
| | | - | - | - |
| | | - | - | - |
| | | - | - | - |
| | | - | - | - |

| | |
|---|---|
| RE = Right Eye | LE = Left Eye |
| - = no opaqueness | + = initial lens opaqueness |
| ++ = evident lens opaqueness | +++ = lens completly affected by cataract |

In brackets the number of animal used is indicated

b) Cataract due to hypergalactosemic diet.

Female albino Sprague - Dowely rats having an average weight of 70 g have been used. Said rats have been fed a diet consisting of 50% galactose and the remaining 50% standard rat food during a 20 days period. The animals have been divided in two groups: the first animal group has been administered PGC (10% in buffer) by instilling one drop twice a day into the conjuctive sac, both sides; the second group, consisting of control animals, has been administered buffer only.

The conditions of the various eye sections :
conjunctiva, cornea, interior chamber, lens and vitreous humor have been evaluated at the slit lamp. The examination has been carried out before the beginning of the treatment and then on the 5th, 10th, 15th and 20th day.

Table 2 shows the results of PGC activity on the cataract induced in rats with hypergalactosemic diet. A protective effect of PGC on the formation of lens opaqueness and the lower percent (60%) of opaque lenses as a whole in the treated animals relative to the controls can be noticed.

TABLE 2

Activity of the dipeptide pyroglutamyl-cysteine (PGC) on the cataract induced by a hypergalactosemic diet

| Progress from treatment beginning (days) | | 0 | 15 | 10 | 15 | 20 |
|---|---|---|---|---|---|---|
| lens opaqueness | | | | | | |
| controls (10) | RE | - | - | - | + | +++ |
| | | - | - | - | ++ | ++ |
| | | - | - | - | ++ | +++ |
| | | - | - | + | ++ | +++ |
| | | - | - | - | + | +++ |
| | | - | - | - | + | ++ |
| | | - | - | + | - | +++ |
| | | - | - | + | + | +++ |
| | | - | - | + | ++ | +++ |
| | | - | - | + | ++ | +++ |
| | LE | - | - | + | + | +++ |
| | | - | - | + | + | +++ |
| | | - | - | - | + | ++ |
| | | - | - | + | ++ | +++ |
| | | - | - | - | ++ | +++ |
| | | - | - | + | ++ | +++ |
| | | - | - | + | + | +++ |
| | | - | - | - | - | ++ |
| | | - | - | - | + | +++ |
| | | - | - | - | + | +++ |
| Treated (10) | RE | - | - | - | - | +++ |
| | | - | - | - | - | ++ |
| | | - | - | - | - | ++ |
| | | - | - | - | + | ++ |
| | | - | - | - | + | ++ |
| | | - | - | - | - | + |
| | | - | - | - | + | + |
| | | - | - | - | - | + |
| | | - | - | - | - | + |
| | | - | - | - | - | + |
| | LE | - | - | - | + | +++ |
| | | - | - | - | - | ++ |
| | | - | - | - | - | ++ |
| | | - | - | - | + | +++ |
| | | - | - | - | + | +++ |
| | | - | - | - | + | ++ |
| | | - | - | - | - | + |
| | | - | - | - | - | + |
| | | - | - | - | + | ++ |
| | | - | - | - | - | + |

RE = Right Eye

LE = Left Eye

- = no opaqueness

+ = initial lens opaqueness

++ = evident lens opaqueness

+++ = lens completly affected by cataract

In brackets the number of animals used is indicated

c) Experimental model of in vitro cataract.

Male albino Wistar rats having an average weight of 180 g have been used. After decapitation, both eye bulbs have been enucleated and the lenses have been removed from the back. The isolated lenses have been incubated at 37°C during 5 days in culture broth 199 containing 15% of foetal bovine serum (Laboratorio Gebco) and a 55.5 mM concentration of glucose. PGC has been added at a 1M concentration into the culture broth of only some lenses. The observations have been carried out at the 3rd and 5th day with the use of a stereo microscope and a Nikon camera with annular flash.

Table 3 shows the results of the induction of cataract in vitro into the rat lenses. It can be observed that in the control lenses the opaqueness development begins after only 72 hours of incubation and is complete after 120 hours. In the lenses treated with PGC low levels of opaqueness can not be observed before 120 hours of treatment.

TABLE 3

| Activity of the dipeptide pyroglutamyl-cysteine (PGC) on the cataract induced in vitro. | | |
|---|---|---|
| Progress from treatment beginning (hours) | | |
| | 72 | 120 |
| lens opaqueness controls (8) | + +<br>+ +<br>+<br>+<br>+ +<br>+<br>+<br>+ + | + + +<br>+ + +<br>+ +<br>+ + +<br>+ +<br>+<br>+ + +<br>+ + + |
| Treated (8) | –<br>–<br>–<br>–<br>–<br>–<br>–<br>– | +<br>+<br>+<br>–<br>–<br>+<br>–<br>– |
| – = no opaqueness<br>+ = initial lens opaqueness<br>+ + = evident lens opaqueness<br>+ + + = lens completly affected by cataract<br>In brackets the number of lenses used is indicated | | |

TOXICITY

The general and local toxicity of PGC has been tested in rabbit and rat after application into the conjunctive sac, intraocular injection and intraperitoneal injection.

The tests have been carried out on male New-Zealand rabbits with an average weight of about 1.5 kgs and in male Wistar rats with an average weight of 180 g, in appearingly good health.

A- INSTILLATION INTO THE CONJUNCTIVE SAC

The toxicity of PGC administered by conjunctive instillation has been evaluated after acute and sub-acute (20 days) administration. To this purpose, 12 rabbits have been used and have been subjected to instillation of a PGC-containing (10%) collyrium (2 drops, 3 times a day). The treatment has been carried on during 20 days. During the treatment the animals have been checked every day to detect the incidental

mortality, the general state, the spontaneous behaviour and the food consumption. At the beginning of the treatment and at 10 days intervals thereafter the body weight of the animals was measured. Before the experiment and after the last application the treated animals and an equal number of non-treated animals have been placed during 12 hours in methabolism measurement cages, where the urine was collected and proteines, hemoglobine, ketone bodies and bilirubine were detected by means of Labstix tests strips. At the end of the treatment, two hours after the last application, blood has been drawn from the animals in the necessary amount for the hematologic determinations. The topical tolerability of the preparation has been studied by means of an ophthalmological examination that has been carried out every second day during the treatment. The following parameters have been observed at the slit lamp:

- conjunctive condition;
- corneal epythelium condition;
- anterior chamber condition and possible presence of opaqueness;
- back ground condition;

12 hours after the last application, the animals have been sacrificed under phenobarbital anestesy and the eyes have been removed together a portion of conjunctive. The pieces have been used for the hystological examination.

B - INTRAPERITONEAL INJECTION

The systemic tolerability of PGC has been studied also after acute administration by means of intraperitoneal injection.

10 rabbits have received the substance (400 mg/kg) injected intraperitoneally.

24 hours after the administration of the product, the following examination have been carried out on urine and blood:

- blood cell (red and white) count
- determination of glycemia and acotemia;
- evaluation of SGOT and SGPT transaminases

In table 4 the results relative to the body-weight increase of the rabbits treated with PGC both by instillation and intraperitoneally are reported. No growth modification has been observed in the treated animals relative to the control ones.

Also the urine and blood examination have not shown any changes of the hematological or hematochemical constants or of the urine composition (table 5).

No phlogosis symptom has been observed in the animal treated at the conjuncting level. This absence of phlogosis has been confirmed by the hystological examination of the conjunctive, the cornea, the iris, the uvea and the retina.

TABLE 4

| Body weight increase in rabbits treated with PGC. | | | |
|---|---|---|---|
| TREATMENT | BODY WEIGHT INCREASE (KG) ON DAY | | |
| | 0 | 20 | (n) |
| 1) PGC, 10% collyrium | 1.60±0.08 | 2.34±0.12 | (12) |
| 2) Saline solution | 1.52±0.07 | 2.36±0.10 | (12) |
| 3) PGC intraperitoneally | 1.45±0.05 | 2.25±0.08 | (10) |
| 4) Saline solution | 1.50±0.10 | 2.32±0.11 | (10) |
| The values shown are average values ± SD. In brackets the number of animals. | | | |

TABLE 5

Haematological and haematochemical constans in rabbits treated with PGC.

| Treatment | RBC (x10) | WBC (x10) | glycemia mg% | azotemia mg% | SGOT mU/ml | SGPT mU/ml | (n) |
|---|---|---|---|---|---|---|---|
| 1) PGC (instill.) | 5.03 | 7.82 | 96.4 | 28.0 | 15.78 | 10.2 | (12) |
| 2) Saline solution (instill.) | 5.05 | 7.78 | 98.2 | 27.3 | 16.0 | 11.1 | (12) |
| 3) PGC (i.p.) | 5.00 | 7.80 | 101.2 | 28.5 | 15.9 | 10.5 | (10) |
| 4) Saline solution (i.p.) | 5.03 | 7.85 | 100.6 | 28.3 | 16.1 | 10.4 | (10) |

The values are average values. In brackets the number of animals.

**Claims**

**Claims for the following Contracting States : AT, BE, CH, DE, FR, GB, IT, LI, LU, NL, SE**

1. The dipeptide L-pyroglutamyl-L-cysteine of formula

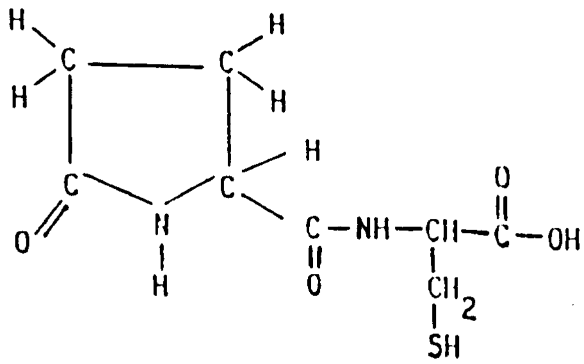

for use as an active therapeutic substance.

2. The dipeptide according to claim 1 for use as substance having anticataractogenic activity.

3. A medical preparation containing the dipeptide of claim 1 and a pharmaceutically inert excipient.

4. A substance having anticataractogenic activity containing a dipeptide as claimed in claim 1 and a pharmaceutically inert excipient.

**Claims for the following Contracting States : ES, GR**

1. Method for the preparation of a therapeutic substance, characterized in that it comprises admixing the dipeptide L-pyroglutamyl-L-cysteine of formula

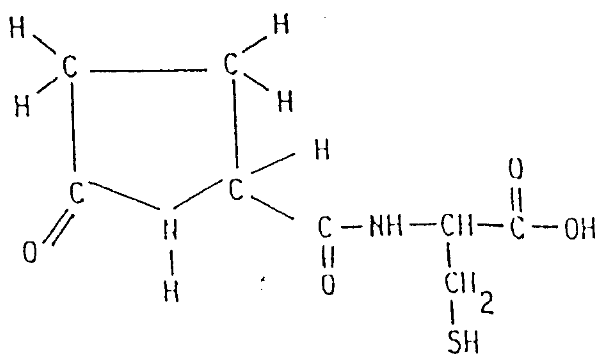

with a pharmaceutically acceptable excipient.

2. Method for the preparation of a therapeutic substance according to claim 1, characterized in that said substance has an anticataractogenic activity.

3. Method for the preparation of a therapeutic substance according to claims 1 and 2 characterized in that said substance is for use in a medical preparation.

4. Method for the preparation of a therapeutic substance according to claims 1 to 3, characterized in that said excipient is a pharmaceutically inert excipient.

## Patentansprüche

**Patentansprüche für folgende Vertragsstaaten : AT, BE, CH, DE, FR, GB, IT, LI, LU, NL, SE**

1. Das Dipeptid L-Pyroglutamyl-L-cystein der Formel

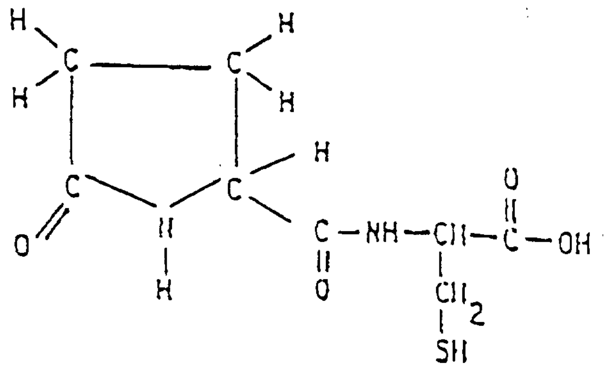

für die Verwendung als aktive therapeutische Substanz.

2. Das Dipeptid nach Anspruch 1 für die Verwendung als Substanz mit antikataraktogenischer Aktivität.

3. Medizinisches Präparat, dadurch **gekennzeichnet,** daß es das Dipeptid nach Anspruch 1 und einen pharmazeutisch inerten Arzneimittelträgerstoff enthält.

4. Substanz mit antikataraktogenischer Aktivität, dadurch **gekennzeichnet,** daß sie ein Dipeptid nach Anspruch 1 und einen pharmazeutisch inerten Arzneimittelträgerstoff enthält.

**Patentansprüche für folgende Vertragsstaaten : ES, GR**

1. Verfahren zur Herstellung einer therapeutischen Substanz, dadurch **gekennzeichnet,** daß das Dipeptid L-Pyroglutamyl-L-cystein der Formel

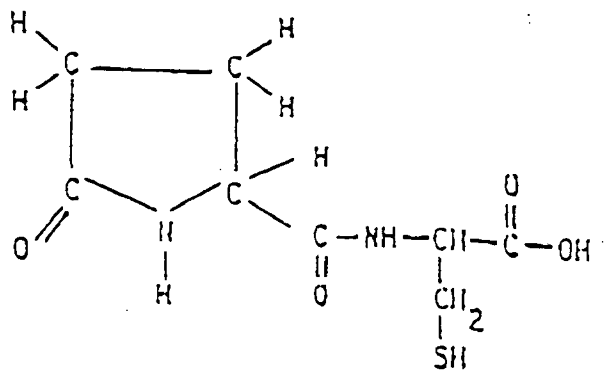

mit einem pharmazeutisch annehmbaren Arzneimittelträgerstoff vermischt wird.

2. Verfahren zur Herstellung einer therapeutischen Substanz nach Anspruch 1, dadurch **gekennzeichnet,** daß die Substanz eine antikataraktogenische Aktivität aufweist.

3. Verfahren zur Herstellung einer therapeutischen Substanz nach einem der Ansprüche 1 und 2, dadurch **gekennzeichnet,** daß die Substanz für die Verwendung in einem medizinischen Präparat bestimmt ist.

4. Verfahren zur Herstellung einer therapeutischen Substanz nach einem der Ansprüche 1 bis 3, dadurch **gekennzeichnet,** daß der Arzneimittelträgerstoff ein pharmazeutisch inerter Arzneimittelträgerstoff ist.

## Revendications

### Revendications pour les Etats contractants suivants : AT, BE, CH, DE, FR, GB, IT, LI, LU, NL, SE

**1.** Le dipeptide L – pyroglutamyl – L – cystéine, représenté par la formule :

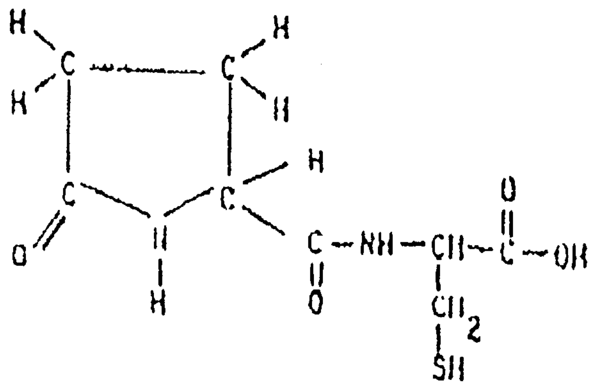

destiné à être utilisé comme substance active thérapeutique,

**2.** Dipeptide selon la revendication 1, destiné à être utilisé comme substance ayant une activité anticataractogène.

**3.** Préparation médicamenteuse contenant le dipeptide tel que défini à la revendication 1 et un excipient pharmaceutiquement inerte.

**4.** Substance ayant une activité anticataractogène contenant un dipeptide tel que défini à la revendication 1 et un excipient pharmaceutiquement inerte.

### Revendications pour les Etats contractants suivants : ES, GR

**1.** Procédé de préparation d'une substance thérapeutique, caractérisé par le fait qu'il comprend le mélange du dipeptide L – pyroglutamyl – L – cystéine représenté par la formule :

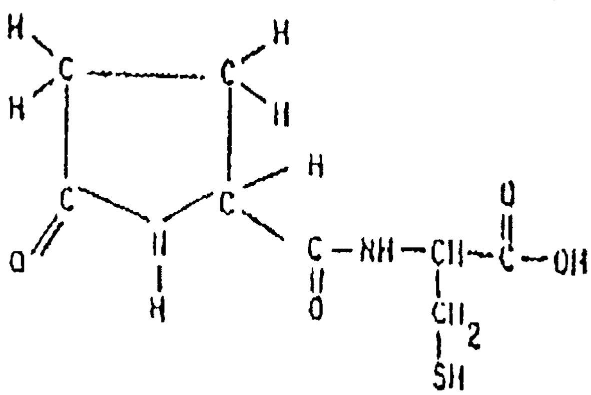

avec un excipient pharmaceutiquement acceptable.

**2.** Procédé de fabrication d'une substance thérapeutique selon la revendication 1, caractérisé par le fait que ladite substance présente une activité anticataractogène.

**3.** Procédé de fabrication d'une substance thérapeutique selon l'une des revendications 1 et 2, caractérisé par le fait que ladite substance est destinée à être utilisée dans une préparation médicamenteuse.

4. Procédé de fabrication d'une substance thérapeutique selon l'une des revendications 1 à 3, caractérisé par le fait que ledit excipient est un excipient pharmaceutiquement inerte.